# FASCICULE DE BREVET EUROPEEN

(11) **EP 2 293 888 B1**
(45) Date de publication et mention de la délivrance du brevet: **22.07.2015**
(21) Numéro de dépôt: 09757734.0
(22) Date de dépôt: 26.05.2009
(51) Int. Cl.: C12M 1/107, C12M 1/00, C02F 11/04, C12P 5/02

(54) **PROCEDE ET INSTALLATION DE TRAITEMENT DE DECHETS ET DE PRODUCTION DE METHANE**
VERFAHREN UND ANLAGE ZUR VERARBEITUNG VON ABFALL UND ERZEUGUNG VON METHAN
METHOD AND PLANT FOR PROCESSING WASTE AND PRODUCING METHANE

(30) Priorité: 05.06.2008 FR 0853724
(43) Date de publication de la demande: 16.03.2011
(73) Titulaire: Veolia Propreté, 92000 Nanterre (FR)
(72) Inventeur: CACHO, Jesùs, F-78200 Mantes La Jolie (FR); CAUDART, Olivier, F-77181 Courtry (FR); LAGIER, Thomas, F-78270 Blaru (FR); PESLERBE, Pascal, F-60260 Lamorlaye (FR); VIGNERON, Vassilia, F-78130 Les Mureaux (FR)
(74) Mandataire: Novagraaf Technologies
(86) Numéro de dépôt international: PCT/FR2009/050976
(87) Numéro de publication internationale: WO 2009/147358

(56) Documents cités:
- EP-A- 0 810 041
- FR-A- 2 812 570
- US-A1- 2008 193 994

## Description

La présente invention concerne, de façon générale, le domaine du traitement et de la valorisation de déchets.

Plus précisément, l'invention concerne, selon un premier de ses aspects, un procédé de traitement de déchets et de production de méthane. Ce procédé comprend une opération initiale de remplissage d'au moins une enceinte avec lesdits déchets, et une opération de dégradation anaérobie des déchets dans au moins une enceinte remplie, au cours de laquelle du méthane est produit.

Un tel procédé est notamment connu par l'exemple qu'en donne le document FR 2 812 570.

Le procédé décrit dans le document FR 2 812 570 comprend plusieurs étapes dont notamment une préparation des déchets par broyage, l'enfouissement des déchets dans une cellule anaérobie, le contrôle de l'humidité et de la température. La cellule est ensuite réutilisée après réouverture et les déchets subissent un post-traitement afin d'être valorisés. Un seul type de réacteur, ici la cellule anaérobie, est utilisé. Ce procédé ne permet de traiter que des déchets de qualité similaire, ici les ordures ménagères et les déchets industriels banals.

On connaît le stockage de déchets tel qu'en centre de stockage. Le stockage accepte tous les déchets tels qu'ils sont, dans un grand volume dans lequel leur dégradation se fait naturellement. Toutefois, ce mode de traitement de déchets présente l'inconvénient d'être long dans le temps, en général au moins d'une durée de trente ans.

On connaît aussi des procédés de méthanisation de déchets, dans lesquels la dégradation des déchets est activée, notamment par l'apport de micro-organismes, afin qu'elle se fasse plus rapidement que dans un centre de stockage. Mais ce mode de traitement de déchets ne permet pas de traiter tous les déchets.

Dans ce contexte, la présente invention a pour but de proposer un procédé exempt de l'une au moins des limitations précédemment évoquées, et notamment un procédé permettant une production de méthane plus efficace.

A cette fin, le procédé de l'invention, par ailleurs conforme à la définition générique qu'en donne le préambule ci-dessus, est essentiellement caractérisé en ce que :
- lors de l'opération initiale de remplissage, au moins une grande enceinte et au moins une petite enceinte sont remplies respectivement de déchets faiblement organiques et de déchets fortement organiques, une grande enceinte présentant une contenance de volume au moins vingt-cinq (25) fois supérieur au volume de contenance d'une petite enceinte,
- les déchets faiblement organiques, tels que des ordures ménagères et/ou des déchets industriels banals, présentent une charge organique plus faible que les déchets fortement organiques, qui sont dégradables en moins de trois ans, lesdits déchets fortement organiques étant de préférence solides, et comprenant par exemple l'un au moins des déchets choisis parmi les boues de station d'épuration des eaux résiduaires urbaines, une fraction fermentescible d'ordures ménagères, des résidus organiques de traitements de déchets, des déchets alimentaires, des déchets de jardin, des résidus de l'industrie agroalimentaire, et des résidus de l'agronomie, et
- pendant l'opération de dégradation, une fraction liquide générée dans au moins une grande enceinte est introduite dans au moins une petite enceinte.

L'invention permet de dégrader de façon accélérée la matière organique de déchets, tout en produisant du méthane afin de le valoriser en énergie. Pour cela, l'invention s'appuie sur une synergie entre des bioréacteurs contenant différentes qualités de déchets.

On entend par bioréacteur une enceinte dédiée à la dégradation de déchets.

On entend par dégradation de déchets une décomposition de la matière organique qu'ils contiennent, due à une action biologique notamment de micro-organismes, d'enzymes, et/ou de champignons. Selon la définition de l'invention, des termes équivalents au terme « dégradation » peuvent être « digestion » ou « fermentation » ou « biodégradation ».

Un déchet dégradé, dans la présente invention, s'entend d'un déchet dont au moins 60% du potentiel biogaz initial ont été obtenus, c'est-à-dire d'un déchet qui a produit 60% du biogaz pouvant être obtenu en conditions optimales de laboratoire, ou s'entend d'un déchet dont au moins 60% du carbone solubilisable initial ont été obtenus, c'est-à-dire d'un déchet qui a perdu au moins 60% du carbone pouvant être perdu en conditions optimales de laboratoire.

Dans l'invention, la dégradation des déchets a lieu en l'absence d'oxygène. C'est donc une dégradation anaérobie.

Le procédé de l'invention permet notamment d'être plus flexible qu'un procédé simple de méthanisation hors sol et plus rapide qu'un procédé de stockage classique.

Le procédé de l'invention présente l'avantage d'obtenir l'accélération de la dégradation des déchets et par conséquent l'accélération de la production de biogaz, et donc de méthane valorisable.

Selon une version préférentielle de l'invention, une fraction liquide générée dans au moins une petite enceinte est introduite dans au moins une grande enceinte.

De manière avantageuse, l'opération initiale de remplissage n'est précédée d'aucun traitement mécanique des déchets.

Classiquement, certaines enceintes sont réutilisables, ou autrement dit réversibles, c'est-à-dire que, dans ces enceintes, une excavation des déchets a lieu, ce qui permet de les réutiliser pour y dégrader de nouveaux déchets.

Les durées de traitement dans ces enceintes réversibles dépendent de la qualité du déchet entrant, qui dans l'invention est choisi tel que sa dégradation dans une petite enceinte selon l'invention ne dépasse pas une durée de trois ans.

D'autres enceintes sont dites « irréversibles » ou « fixes ». Une fois de telles enceintes remplies de déchets, elles ne sont pas ré-ouvertes pour en extraire les déchets dégradés.

Dans l'invention, de préférence, au moins une grande enceinte est irréversible alors qu'au moins une petite enceinte est réversible.

Une grande enceinte est par exemple un casier d'installation de stockage de déchets, et plus particulièrement un casier d'installation de stockage de déchets non dangereux (ISDND).

Ainsi, l'invention propose un couplage entre une enceinte, telle qu'une cuve de réacteur de méthanisation, et un casier d'ISDND, ce couplage permettant notamment de traiter des déchets qui ne pourraient pas l'être directement dans une cuve de méthanisation classique.

Le procédé selon l'invention peut comprendre en outre une opération finale d'excavation des déchets dégradés d'au moins une petite enceinte.

Cette excavation des déchets permet éventuellement de mettre en place une ou plusieurs opérations de valorisation de la matière correspondant aux déchets dégradés, telles que l'utilisation comme combustible solide de substitution, une valorisation agronomique, ou un recyclage, et permet également de s'inscrire dans une politique de développement durable en réutilisant l'enceinte pour le traitement de nouveaux déchets.

Un des buts de l'invention est d'augmenter la teneur en eau des déchets et de les ensemencer, afin d'optimiser leur dégradation.

Dans ce but, les enceintes peuvent être ensemencées par des fractions liquides, ou lixiviats, qu'elles génèrent, et/ou par les fractions liquides provenant d'au moins une autre enceinte.

En complément de ces ensemencements par le biais des fractions liquides, le procédé de l'invention peut comprendre, de préférence pendant l'opération initiale de remplissage, une opération d'ensemencement consistant à introduire, dans au moins une petite enceinte et/ou au moins une grande enceinte, des micro-organismes, des champignons, et/ou des enzymes, afin qu'ils participent à la dégradation des déchets.

Dans un mode de réalisation particulier de l'invention, au moins une enceinte en milieu liquide est remplie, de manière continue ou discontinue, de déchets fortement organiques liquides, tels que des effluents liquides industriels issus de l'agroalimentaire, lesdits déchets liquides subissant, dans au moins une enceinte en milieu liquide, l'opération de dégradation anaérobie, une grande enceinte présentant une contenance de volume au moins 25 fois supérieur au volume de contenance d'une enceinte en milieu liquide ; et la fraction liquide générée dans au moins une grande enceinte et/ou la fraction liquide générée dans au moins une petite enceinte est introduite dans au moins une enceinte en milieu liquide.

De manière avantageuse, une fraction liquide générée dans au moins une enceinte en milieu liquide par l'opération de dégradation anaérobie est introduite dans au moins une grande enceinte et/ou dans au moins une petite enceinte.

Une enceinte en milieu liquide selon l'invention est de préférence hors sol.

Dans l'invention, les enceintes sont étanches. Notamment, l'oxygène n'y pénètre pas.

Le procédé selon l'invention peut comprendre en outre une opération de prétraitement consistant à ce qu'au moins une des fractions liquides générées respectivement dans au moins une grande enceinte, dans au moins une petite enceinte, et dans au moins une enceinte en milieu liquide, soit traitée avant d'être introduite dans l'une quelconque de ces enceintes, notamment par traitement thermique, par nitrification, par ajout de tampon, par ajout de micro-organismes, par ajout d'enzymes, et/ou par ajout de champignons.

L'invention concerne, selon un second de ses aspects, une installation pour le traitement de déchets et la production de méthane, adaptée à la mise en oeuvre du procédé selon l'invention. Cette installation comprend :
- au moins une grande enceinte adaptée à recevoir des déchets faiblement organiques, telle qu'un casier d'installation de stockage de déchets non dangereux, et au moins une petite enceinte adaptée à recevoir des déchets fortement organiques, une grande enceinte présentant une contenance de volume au moins 25 fois supérieur au volume de contenance d'une petite enceinte, et lesdits déchets faiblement organiques présentant une charge organique plus faible que les déchets fortement organiques qui sont dégradables en moins de trois ans,
- des moyens d'introduction d'une fraction liquide issue d'au moins une grande enceinte dans au moins une petite enceinte, et de préférence aussi
- des moyens d'introduction d'une fraction liquide issue d'au moins une petite enceinte dans au moins une grande enceinte.

Selon un mode particulier de l'invention, au moins une petite enceinte est adaptée à recevoir des déchets fortement organiques solides, et l'installation comprend en outre :
- au moins une enceinte en milieu liquide adaptée à recevoir des déchets fortement organiques liquides, une grande enceinte présentant une contenance de volume au moins 25 fois supérieur au volume de contenance d'une enceinte en milieu liquide, et
- des moyens d'introduction d'une fraction liquide issue d'au moins une grande enceinte et/ou d'une fraction liquide issue d'au moins une petite enceinte dans au moins une enceinte en milieu liquide.

De manière avantageuse, l'installation selon l'invention comprend en outre des moyens d'introduction d'une fraction liquide d'au moins une enceinte en milieu liquide vers au moins une grande enceinte et/ou vers au moins une petite enceinte.

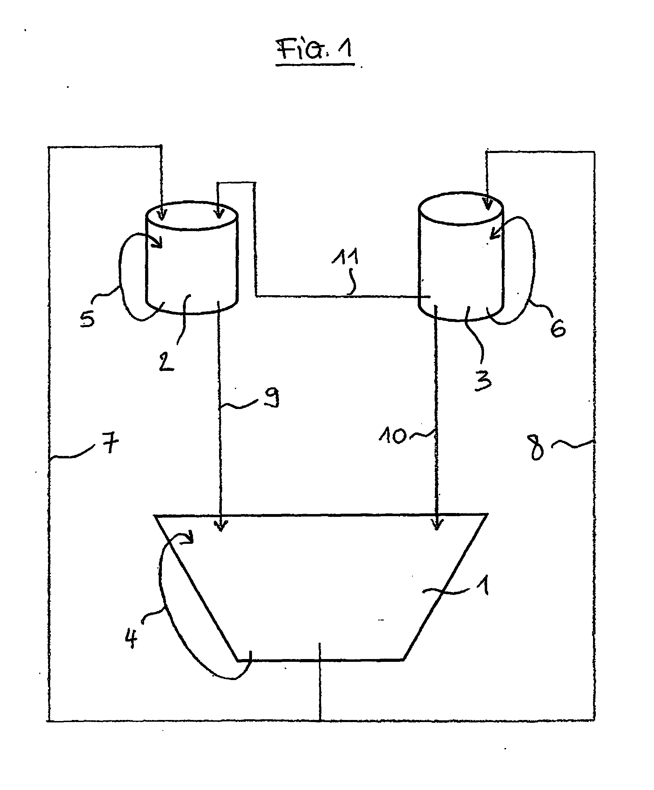

D'autres caractéristiques et avantages de l'invention ressortiront clairement de la description détaillée qui en est faite ci-après, à titre indicatif et nullement limitatif, en référence à la figure 1 annexée représentant schématiquement une installation selon l'invention, mettant en oeuvre le procédé selon l'invention.

L'installation représentée à la figure 1 a subi une opération initiale de remplissage de plusieurs enceintes 1, 2, 3 avec des déchets n'ayant pas subi au préalable de traitement mécanique.

En effet, contrairement aux procédés classiques connus, dans l'invention, les déchets peuvent être directement mis en place avec une éventuelle sélection au préalable qui se fait uniquement sur l'origine des déchets, mais sans traitement mécanique.

Le remplissage est accompagné ou non d'un ensemencement.

On entend par ensemencement un apport de micro-organismes et/ou autres molécules ou organismes capables d'accélérer la dégradation des déchets.

Une caractérisation des déchets peut être réalisée également pendant cette opération de remplissage. On peut notamment mettre en place une identification de la nature des déchets, un relevé du taux d'humidité et/ou du taux de matières volatiles, et/ou une évaluation du potentiel de production de méthane. Par exemple, les déchets incluent des déchets verts, des fruits, des légumes, de la viande, du bois tel que des palettes, du plastique, du papier, du carton, des boues, et/ou des textiles.

Une fois les enceintes 1, 2, 3 remplies, les déchets y subissent une dégradation en conditions anaérobies, soit sans oxygène, et à une température de préférence comprise entre 30 et 60°C, et tout préférentiellement à une température de 35°C.

La vitesse de dégradation des déchets est augmentée au moyen de l'injection des fractions liquides provenant des trois types 1, 2, 3 d'enceinte. Une optimisation de la production de méthane est ainsi obtenue.

Dans la figure 1, l'installation inclut une grande enceinte 1, une petite enceinte 2, et une enceinte 3 en milieu liquide.

La grande enceinte 1 est une installation permanente comme une ISDND de type bioréacteur. Elle est placée dans le sol.

La petite enceinte 2, hors-sol, est une cuve de méthanisation, aussi appelée un digesteur. Selon l'invention, une petite enceinte peut autrement être une cellule étanche dans le sol.

L'enceinte 3 en milieu liquide est une cuve de réaction hors-sol.

La grande enceinte 1 présente une contenance de volume au moins 25 fois supérieur au volume de contenance de la petite enceinte 2, ainsi qu'au volume de contenance de l'enceinte 3 en milieu liquide.

Par exemple, la petite enceinte 2 et/ou l'enceinte 3 en milieu liquide présentent une contenance de volume compris entre 500 et 3000 mètres cube (m³), de préférence entre 1000 et 2000 m³.

La grande enceinte 1 présente par exemple une contenance de volume compris entre 80000 m³ et 4000000 m³.

Le procédé et l'installation selon l'invention permettent de traiter des déchets d'origines diverses.

Dans la grande enceinte 1, des déchets habituellement reçus sur une ISDND sont initialement introduits puis dégradés. Ces déchets sont notamment des ordures ménagères et/ou des déchets industriels banals (DIB).

Dans la petite enceinte 2, sont introduits et dégradés des déchets riches en composés organiques, tels que des boues, la fraction fermentescible des ordures ménagères (FFOM), des résidus organiques de traitements de déchets, ou tout autre déchet contenant une fraction organique importante parmi notamment les déchets alimentaires, les déchets fermentescibles, et les déchets de jardin.

Les déchets initialement introduits dans l'enceinte 2 sont de préférence dépourvus de matériaux tels que bois, textile, papier, et carton, ou n'en contiennent qu'une quantité réduite.

Les déchets de l'enceinte 2 sont dégradables en moins de trois ans, ou de préférence en moins d'un an.

Enfin, des déchets liquides présentant une fraction organique importante, issus par exemple de l'industrie, en particulier de l'industrie agroalimentaire, sont introduits et dégradés dans l'enceinte 3 en milieu liquide.

Cette enceinte 3 est alimentée, en continu ou en discontinu, avec des déchets liquides. Lorsque le liquide en sortie d'enceinte 3, après dégradation des déchets, atteint les normes de rejet imposées par la législation en vigueur, il peut être évacué pour être remplacé par un volume équivalent de nouveaux déchets liquides. Le temps de séjour des déchets dans l'enceinte 3 dépend de leur qualité en entrée.

Les enceintes 1, 2 et 3 ainsi remplies de déchets libèrent chacune du méthane qui peut alors être récupéré et valorisé.

L'avantage du stockage de déchets, tel que dans l'enceinte 1, est d'accepter tous les déchets tels qu'ils sont, soit sans prétraitement, dans un grand volume dans lequel la dégradation se fait naturellement. Toutefois, ce mode de traitement de déchets présente l'inconvénient d'être long dans le temps.

L'avantage de la méthanisation de déchets, telle que dans l'enceinte 2, est principalement d'activer la dégradation de ces déchets pour qu'elle se fasse plus rapidement que dans les centres de stockage. Mais ce mode de traitement de déchets ne permet pas de traiter tous les déchets.

Le procédé de l'invention est plus efficace que les procédés connus en ce qu'il offre une synergie entre le stockage de déchets, notamment dans l'enceinte 1, et une méthanisation de déchets, notamment dans l'enceinte 2.

En outre, l'invention propose une utilisation avantageuse des fractions liquides issues des différentes enceintes 1, 2, 3.

En effet, une fraction liquide issue d'une enceinte est injectée dans une autre enceinte ou dans cette même enceinte. Ces injections de fraction liquide permettent en conséquence d'augmenter la teneur en eau des déchets dans les enceintes 1, 2, 3, et permettent une circulation de matière organique et/ou un ensemencement des déchets.

Les différents flux d'injection de ces fractions liquides ou lixiviats sont les suivants :
- flux 4, 5, et 6 : injection de la fraction liquide issue d'une enceinte 1, 2, ou 3 dans cette même enceinte 1, 2, ou 3 ;
- flux 7 et 8 : injection de la fraction liquide issue de la grande enceinte 1 dans la petite enceinte 2 et/ou dans l'enceinte 3 en milieu liquide ; ces flux 7, 8 optimisent non seulement l'utilisation des lixiviats, générés par la grande enceinte 1, en augmentant la teneur en eau des déchets traités dans la petite enceinte 2 et/ou dans l'enceinte 3 en milieu liquide, mais aussi la dégradation du carbone organique dissous dans ces lixiviats ;
- flux 9 : injection de la fraction liquide issue de la petite enceinte 2 dans la grande enceinte 1 ;
- flux 10 et 11 : injection de la fraction liquide issue de l'enceinte 3 en milieu liquide dans la grande enceinte 1 et dans la petite enceinte 2.

On peut faire varier le taux d'injection du lixiviat du flux 7 pour obtenir une séparation des phases acidogène et méthanogène dans la petite enceinte 2. Dans ce cas, on facilite le passage de la matière organique des déchets traités dans l'enceinte 2 dans la fraction liquide en sortie d'enceinte 2. Cette fraction, recueillie et analysée, peut ensuite être injectée dans la grande enceinte 1 afin que du méthane y soit produit.

On peut autrement optimiser le taux d'injection d'un lixiviat dans une enceinte de manière à ce que les déchets s'y trouvant aient le temps d'être dégradés jusqu'au stade de production de méthane. Dans ce cas, les flux 7 à 11 sont optimisés pour l'ensemencement en micro-organismes actifs des déchets se trouvant dans l'enceinte d'arrivée du flux.

Un lixiviat peut être soumis à diverses analyses en sortie d'enceinte avant d'être injecté dans une enceinte, afin d'en connaître les caractéristiques physico-chimiques telles que le pH, la température.

En conséquence ou non des résultats de ces analyses, un lixiviat issu d'une enceinte peut être traité avant d'être introduit dans la même ou une autre enceinte. Ce prétraitement peut être, par exemple, une décantation, une nitrification, une oxydation, un ajout de tampon, un ajout de réactif, un chauffage, ou un ensemencement biologique, notamment par des bactéries, des virus, et/ ou des champignons.

L'installation selon l'invention peut par exemple inclure au moins une cuve pour chauffer du lixiviat jusqu'à une température de 35°C, afin que les micro-organismes qu'il contient soient dans des conditions optimales pour dégrader la matière organique des déchets auxquels est ajouté ce lixiviat préalablement chauffé.

On peut aussi mettre en place un capteur de température, dans une petite enceinte notamment, afin de connaître la température au sein des déchets et de pouvoir injecter, après chauffage le cas échéant, une fraction liquide, issue notamment d'une grande enceinte, dont la température permet la dégradation des déchets.

En sortie d'une grande enceinte, on vérifie que, de préférence, la fraction liquide présente un pH au moins égal à 6,8.

Une fois les déchets dégradés dans la petite enceinte 2, une étape d'aération est réalisée avant d'ouvrir l'enceinte 2 pour en excaver les déchets, afin de terminer et/ou stopper la dégradation des déchets.

Les déchets excavés peuvent être alors broyés puis compostés avant séchage. Une partie de ces déchets ainsi traités après excavation peut être utilisée en tant qu'amendement organique, et/ou en tant que combustible de substitution, et une autre partie peut être enfouie dans une installation de stockage de déchets de classe 2 et/ou de classe 3.

L'invention peut faire intervenir plusieurs enceintes de chaque type d'enceinte 1, 2, ou 3.

A titre d'exemple, on peut utiliser treize petites enceintes du type de l'enceinte 2 et dans le sol, et remplir une de ces treize petites enceintes par mois de manière à ce qu'il y ait toujours une enceinte de laquelle les déchets traités sont excavés pour être valorisés. Les fractions liquides issues des treize petites enceintes sont injectées dans une grande enceinte du type de l'enceinte 1, soit dans un casier d'ISDND gérée en mode bioréacteur. La fraction liquide issue de ce casier est également injectée dans les petites enceintes.

L'invention présente encore les avantages suivants par rapport aux procédés existants :
- ne pas nécessiter de prétraitement des déchets avant remplissage d'une enceinte ;
- flexibilité au niveau de la dégradation anaérobie, en fonction du déchet et de la voie métabolique qui se met en place dans le bioréacteur réversible (phase acidogène uniquement ou combinaison des deux phases acidogène et méthanogène), ce qui est possible grâce à la combinaison d'un bioréacteur réversible de petite taille et d'un bioréacteur irréversible d'une taille au moins 25 fois plus grande ; et
- gestion des déchets et de leurs sous-produits dans un même lieu, évitant ainsi d'avoir recours à un transport, et donc permettant un gain de temps et une limitation de la pollution générée lors du transport, notamment par la libération de gaz à effet de serre.

## Revendications

1. Procédé de traitement de déchets et de production de méthane, ledit procédé comprenant une opération initiale de remplissage d'au moins une enceinte avec lesdits déchets, et une opération de dégradation anaérobie des déchets dans au moins une enceinte remplie, au cours de laquelle du méthane est produit, ledit procédé étant **caractérisé en ce que** :
- lors de l'opération initiale de remplissage, au moins une grande enceinte (1) et au moins une petite enceinte (2) sont remplies respectivement de déchets faiblement organiques et de déchets fortement organiques, une grande enceinte (1) présentant une contenance de volume au moins 25 fois supérieur au volume de contenance d'une petite enceinte (2),
- les déchets faiblement organiques, tels que des ordures ménagères et/ou des déchets industriels banals, présentent une charge organique plus faible que les déchets fortement organiques, qui sont dégradables en moins de trois ans, lesdits déchets fortement organiques étant de préférence solides, et comprenant par exemple l'un au moins des déchets choisis parmi les boues de station d'épuration des eaux résiduaires urbaines, une fraction fermentescible d'ordures ménagères, des résidus organiques de traitements de déchets, des déchets alimentaires, des déchets de jardin, des résidus de l'industrie agroalimentaire, et des résidus de l'agronomie, et
- pendant l'opération de dégradation, une fraction liquide générée dans au moins une grande enceinte (1) est introduite dans au moins une petite enceinte (2).

2. Procédé selon la revendication 1, dans lequel une fraction liquide générée dans au moins une petite enceinte (2) est introduite dans au moins une grande enceinte (1).

3. Procédé selon la revendication 1 ou 2, dans lequel l'opération initiale de remplissage n'est précédée d'aucun traitement mécanique des déchets.

4. Procédé selon l'une quelconque des revendications 1 à 3, comprenant en outre une opération finale d'excavation des déchets dégradés d'au moins une petite enceinte (2).

5. Procédé selon l'une quelconque des revendications 1 à 4, comprenant en outre, de préférence pendant l'opération initiale de remplissage, une opération d'ensemencement consistant à introduire, dans au moins une petite enceinte (2) et/ou au moins une grande enceinte (1), des micro-organismes, des champignons, et/ou des enzymes, afin qu'ils participent à la dégradation anaérobie des déchets.

6. Procédé selon l'une quelconque des revendications 1 à 5, dans lequel une grande enceinte (1) est un casier d'installation de stockage de déchets non dangereux.

7. Procédé selon l'une quelconque des revendications 1 à 6, dans lequel au moins une enceinte (3) en milieu liquide est remplie, de manière continue ou discontinue, de déchets fortement organiques liquides, tels que des effluents liquides industriels issus de l'agroalimentaire, lesdits déchets liquides subissant, dans au moins une enceinte (3) en milieu liquide, l'opération de dégradation anaérobie, une grande enceinte (1) présentant une contenance de volume au moins 25 fois supérieur au volume de contenance d'une enceinte (3) en milieu liquide ; et dans lequel la fraction liquide générée dans au moins une grande enceinte (1) et/ou la fraction liquide générée dans au moins une petite enceinte (2)est introduite dans au moins une enceinte (3) en milieu liquide.

8. Procédé selon la revendication 7, dans lequel une fraction liquide générée dans au moins une enceinte (3) en milieu liquide par l'opération de dégradation anaérobie est introduite dans au moins une grande enceinte (1) et/ou dans au moins une petite enceinte (2).

9. Procédé selon l'une quelconque des revendications 1 à 8, comprenant en outre une opération de prétraitement consistant à ce qu'au moins une des fractions liquides générées respectivement dans au moins une grande enceinte (1), dans au moins une petite enceinte (2), et dans au moins une enceinte (3) en milieu liquide, soit traitée avant d'être introduite dans l'une quelconque de ces enceintes (1, 2, 3), notamment par traitement thermique, par nitrification, par ajout de tampon, par ajout de micro-organismes, par ajout d'enzymes, et/ou par ajout de champignons.

10. Installation pour le traitement de déchets et la production de méthane, adaptée à la mise en oeuvre du procédé tel que décrit dans l'une quelconque des revendications 1 à 9, ladite installation comprenant :
- au moins une grande enceinte (1) adaptée à recevoir des déchets faiblement organiques, telle qu'un casier d'installation de stockage de déchets non dangereux, et au moins une petite enceinte (2) adaptée à recevoir des déchets fortement organiques, une grande enceinte (1) présentant une contenance de volume au moins 25 fois supérieur au volume de contenance d'une petite enceinte (2), et lesdits déchets faiblement organiques présentant une charge organique plus faible que les déchets fortement organiques qui sont dégradables en moins de trois ans, et
- des moyens d'introduction d'une fraction liquide issue d'au moins une grande enceinte (1) dans au moins une petite enceinte (2).

11. Installation selon la revendication 10, comprenant en outre des moyens d'introduction d'une fraction liquide issue d'au moins une petite enceinte (2) dans au moins une grande enceinte (1).

12. Installation selon la revendication 10 ou 11, dans laquelle au moins une petite enceinte (2) est adaptée à recevoir des déchets fortement organiques solides, ladite installation comprenant en outre :
- au moins une enceinte (3) en milieu liquide étant adaptée à recevoir des déchets fortement organiques liquides, une grande enceinte (1) présentant une contenance de volume au moins 25 fois supérieur au volume de contenance d'une enceinte (3) en milieu liquide, et
- des moyens d'introduction d'une fraction liquide issue d'au moins une grande enceinte (1) et/ou d'une fraction liquide issue d'au moins une petite enceinte (2) dans au moins une enceinte (3) en milieu liquide.

13. Installation selon la revendication 12, comprenant en outre des moyens d'introduction d'une fraction liquide d'au moins une enceinte (3) en milieu liquide vers au moins une grande enceinte (1) et/ou vers au moins une petite enceinte (2).

## Patentansprüche

1. Verfahren zur Abfallbehandlung und Methanerzeugung, wobei das besagte Verfahren einen ersten Arbeitsgang zum Füllen zumindest eines abgeschlossenen Raumes mit den besagten Abfällen umfasst, und einen Arbeitsgang zum anaeroben Abbau der Abfälle in zumindest einem befüllten abgeschlossenen Raum, im Laufe dessen Methan erzeugt wird, wobei das besagte Verfahren **dadurch gekennzeichnet ist, dass**:
- beim ersten Arbeitsgang zum Füllen zumindest ein großer abgeschlossener Raum (1) und zumindest ein kleiner abgeschlossener Raum (2) jeweils mit schwach organischen Abfällen und stark organischen Abfällen gefüllt werden, wobei der große abgeschlossener Raum (1) einen Inhalt mit einem Volumen aufweist, das mindestens 25 Mal größer ist, als das Volumen des Inhalts des kleinen abgeschlossenen Raumes (2),
- die schwach organischen Abfälle, wie Hausmüll und/ oder allgemeiner Industriemüll eine geringere organische Belastung aufweisen, als die stark organischen Abfälle, die in weniger als drei Jahren abgebaut werden können, wobei die besagten stark organischen Abfälle vorzugsweise feste Abfälle sind, und beispielsweise zumindest einen der Abfälle enthalten, die aus den Klärschlämmen städtischer Abwässer, einer vergärbaren Fraktion aus dem Hausmüll, organischen Rückständen von Abfallbehandlungen, Nahrungsmittelabfällen, Gartenabfällen, Rückständen aus der Nahrungsmittelindustrie und Rückständen aus der Agronomie ausgewählt werden,
und
- während des Abbauvorgangs eine flüssige Fraktion, die in zumindest einem großen abgeschlossenen Raum (1) generiert wird, in zumindest einen kleinen abgeschlossenen Raum (2) eingebracht wird.

2. Verfahren nach Anspruch 1, wobei eine flüssige Fraktion, die in zumindest einem kleinen abgeschlossenen Raum (2) generiert wird, in zumindest einen großen abgeschlossenen Raum (1) eingebracht wird.

3. Verfahren nach Anspruch 1 oder 2, wobei dem ersten Arbeitsgang zum Füllen keine mechanische Behandlung der Abfälle vorangeht.

4. Verfahren nach irgendeinem der Ansprüche 1 bis 3, das darüber hinaus einen letzten Arbeitsgang zum Ausheben der abgebauten Abfälle aus zumindest einem kleinen abgeschlossenen Raum (2) umfasst.

5. Verfahren nach irgendeinem der Ansprüche 1 bis 4, das darüber hinaus und vorzugsweise während des ersten Arbeitsganges zum Füllen, einen Arbeitsgang zum Einsäen umfasst, der darin besteht, in zumindest einen kleinen abgeschlossenen Raum (2) und/ oder zumindest einen großen abgeschlossenen Raum (1) Mikroorganismen, Pilze und / oder Enzyme einzubringen, damit diese am anaeroben Abbau der Abfälle teilhaben.

6. Verfahren nach irgendeinem der Ansprüche 1 bis 5, wobei ein großer abgeschlossener Raum (1) ein Einbaufach zum Lagern ungefährlicher Abfälle ist.

7. Verfahren nach irgendeinem der Ansprüche 1 bis 6, wobei zumindest ein abgeschlossener Raum (3) im flüssigen Milieu durchgehend oder nicht durchgehend mit stark organischen flüssigen Abfällen, wie mit flüssigen Industrieabflüssen aus der Nahrungsmittelindustrie gefüllt wird, wobei die besagten flüssigen Abfälle in zumindest einem abgeschlossenen Raum (3) im flüssigen Milieu den Arbeitsgang des anaeroben Abbaus erfahren, wobei ein großer abgeschlossener Raum (1) einen Inhalt mit einem Volumen aufweist, das mindestens 25 Mal größer ist, als das Volumen des Inhalts eines abgeschlossenen Raumes (3) im flüssigen Milieu; und wobei die flüssige Fraktion, die in zumindest einem großen abgeschlossenen Raum (1) generiert wird, und/ oder die flüssige Fraktion, die in zumindest einem kleinen abgeschlossenen Raum (2) generiert wird, in zumindest einen abgeschlossenen Raum (3) im flüssigen Milieu eingebracht wird.

8. Verfahren nach Anspruch 7, wobei eine flüssige Fraktion, die in zumindest einem abgeschlossenen Raum (3) im flüssigen Milieu durch den Arbeitsgang des anaeroben Abbaus generiert wird, in zumindest einen großen abgeschlossenen Raum (1) und/ oder in zumindest einen kleinen abgeschlossenen Raum (2) eingebracht wird.

9. Verfahren nach irgendeinem der Ansprüche 1 bis 8, das darüber hinaus einen Arbeitsgang zur Vorbehandlung umfasst, der darin besteht, dass zumindest eine der flüssigen Fraktionen, die jeweils in zumindest einem großen abgeschlossenen Raum (1), in zumindest einem kleinen abgeschlossenen Raum (2) und in zumindest einem Raum (3) im flüssigen Milieu generiert wird, behandelt wird, bevor sie in irgendeinen der geschlossenen Räume (1, 2, 3) eingebracht wird, vor allem durch eine thermische Behandlung, durch Salpeterbildung, durch eine Pufferbeigabe, durch die Beigabe von Mikroorganismen, durch die Beigabe von Enzymen, und/ oder durch die Beigabe von Pilzen.

10. Installation zur Abfallbehandlung und zur Methanerzeugung, die sich zur Anwendung des Verfahrens eignet, wie es in irgendeinem der Ansprüche 1 bis 9 beschrieben wird, wobei die besagte Installation folgendes umfasst:
- zumindest einen großen abgeschlossenen Raum (1), der sich dazu eignet, schwach organische Abfälle aufzunehmen, wie ein Einbaufach zum Lagern ungefährlicher Abfälle, und zumindest einen kleinen abgeschlossenen Raum (2), der sich dazu eignet, stark organische Abfälle aufzunehmen, einen großen abgeschlossenen Raum (1), der einen Inhalt mit einem Volumen aufweist, das mindestens 25 Mal größer ist, als das Volumen des Inhalts eines kleinen abgeschlossenen Raumes (2), wobei die besagten schwach organischen Abfälle eine geringere organische Belastung aufweisen, als die stark organischen Abfälle, die in weniger als drei Jahren abgebaut werden können, und
- Mittel zum Einbringen einer flüssigen Fraktion aus zumindest einem großen abgeschlossenen Raum (1) in zumindest einen kleinen abgeschlossenen Raum (2).

11. Installation nach Anspruch 10, die darüber hinaus Mittel zum Einbringen einer flüssigen Fraktion aus zumindest einem kleinen abgeschlossenen Raum (2) in zumindest einen großen abgeschlossenen Raum (1) umfasst.

12. Installation nach Anspruch 10 oder 11, wobei sich zumindest ein kleiner abgeschlossener Raum (2) dazu eignet, feste stark organische Abfälle aufzunehmen, wobei die Installation darüber hinaus folgendes umfasst:
- zumindest einen abgeschlossenen Raum (3) im flüssigen Milieu, der sich dazu eignet, stark organische flüssige Abfälle aufzunehmen, einen großen abgeschlossenen Raum (1), der einen Inhalt mit einem Volumen aufweist, das mindestens 25 Mal größer ist, als das Volumen des Inhalts eines abgeschlossenen Raumes (3) im flüssigen Milieu, und
- Mittel zum Einbringen einer flüssigen Fraktion aus zumindest einem großen abgeschlossenen Raum (1) und / oder einer flüssigen Fraktion aus zumindest einem kleinen abgeschlossenen Raum (2) in zumindest einen abgeschlossenen Raum (3) im flüssigen Milieu.

13. Installation nach Anspruch 12, die darüber hinaus Mittel zum Einbringen einer flüssigen Fraktion aus zumindest einem abgeschlossenen Raum (3) im flüssigen Milieu in zumindest einen großen abgeschlossenen Raum (1) und/ oder einen kleinen abgeschlossenen Raum (2) umfasst.

## Claims

1. Method for processing waste and producing methane, said method comprising an initial filling operation for filling at least one chamber with said waste, and an anaerobic degradation operation for degrading the waste in at least one filled chamber, during which methane is produced, said method being **characterised in that**:
- during the initial filling operation, at least one large chamber (1) and at least one small chamber (2) are respectively filled with slightly organic waste and with highly organic waste, a large chamber (1) having a volume content at least 25 times greater than the content volume of a small chamber (2),
- the slightly organic waste, such as household waste and/or non-hazardous industrial waste, has a lower organic load than the highly organic waste, which can degrade in less than three years, said highly organic waste being preferably solid, and comprising for example at least one of the waste types chosen from urban waste water treatment plant sludge, a fermentable fraction of household waste, organic waste treatment residue, food waste, garden waste, food processing waste and agronomic waste, and
- during the degradation operation, a liquid fraction generated in at least one large chamber (1) is fed into at least one small chamber (2).

2. Method according to claim 1, wherein a liquid fraction generated in at least one small chamber (2) is fed into at least one large chamber (1).

3. Method according to claim 1 or 2, wherein the initial filling operation is not preceded by any mechanical waste treatment process.

4. Method according to any one of claims 1 to 3, further comprising a final excavation operation for excavating the degraded waste from at least one small chamber (2).

5. Method according to any one of claims 1 to 4, further comprising, preferably during the initial filling operation, a seeding operation consisting in feeding, into at least one small chamber (2) and/or at least one large chamber (1), micro-organisms, fungi and/or enzymes so that they take part in the anaerobic waste degradation process.

6. Method according to any one of claims 1 to 5, wherein a large chamber (1) is a non-hazardous waste storage plant compartment.

7. Method according to any one of claims 1 to 6, wherein at least one chamber (3) in a liquid medium is filled, in a continuous or discontinuous manner, with highly organic, liquid waste such as liquid industrial effluents derived from the food processing industry, such liquid waste undergoing, in at least one chamber (3) in a liquid medium, the anaerobic degradation operation, a large chamber (1) having a volume content at least 25 times greater than the content volume of a chamber (3) in a liquid medium; and wherein the liquid fraction generated in at least one large chamber (1) and/or the liquid fraction generated in at least one small chamber (2) is fed into at least one chamber (3) in a liquid medium.

8. Method according to claim 7, wherein a liquid fraction generated in at least one chamber (3) in a liquid medium by the anaerobic degradation operation is fed into at least one large chamber (1) and/or into at least one small chamber (2).

9. Method according to any one of claims 1 to 8, further comprising a pre-treatment operation consisting in that at least one of the liquid fractions respectively generated in at least one large chamber (1), in at least one small chamber (2), and in at least one chamber (3) in a liquid medium, is treated before being fed into any one of these chambers (1, 2, 3), in particular via heat treatment, nitrification, the addition of a buffer, the addition of micro-organisms, the addition of enzymes and/or the addition of fungi.

10. Plant for processing waste and producing methane, designed to implement the method as described in any one of claims 1 to 9, said plant comprising:
- at least one large chamber (1), designed to receive slightly organic waste, such as a non-hazardous waste storage plant compartment, and at least one small chamber (2), designed to receive highly organic waste, a large chamber (1) having a volume content at least 25 times greater than the content volume of a small chamber (2), and said slightly organic waste having an organic load lower than the highly organic waste, which can be degraded in less than three years, and
- means for feeding a liquid fraction derived from at least one large chamber (1) into at least one small chamber (2).

11. Plant according to claim 10, further comprising means for feeding a liquid fraction derived from at least one small chamber (2) into at least one large chamber (1).

12. Plant according to claim 10 or 11, wherein at least one small chamber (2) is designed to receive highly organic, solid waste, said plant further comprising:
- at least one chamber (3) in a liquid medium being designed to receive highly organic, liquid waste, one large chamber (1) having a volume content at least 25 times greater than the content volume of a chamber (3) in a liquid medium, and
- means for feeding a liquid fraction derived from at least one large chamber (1) and/or a liquid fraction derived from at least one small chamber (2) into at least one chamber (3) in a liquid medium.

13. Plant according to claim 12, further comprising means for feeding a liquid fraction from at least one chamber (3) in a liquid medium into at least one large chamber (1) and/or into at least one small chamber (2).
